# EUROPEAN PATENT SPECIFICATION

(11) **EP 2 014 247 B1**
(45) Date of publication and mention of the grant of the patent: **15.12.2010**
(21) Application number: 07013815.1
(22) Date of filing: 13.07.2007
(51) Int. Cl.: A61B 17/86, A61B 17/88

(54) **Device for fixation of bone fractures**
Vorrichtung zur Fixierung von Knochenfrakturen
Dispositif pour la fixation de fractures osseuses

(43) Date of publication of application: 14.01.2009
(73) Proprietor: Stryker Trauma GmbH, 24232 Schönkirchen/Kiel (DE)
(72) Inventor: Dorawa, Klaus, 24232 Schönkirchen (DE); Schwager, Manuel, 8057 Zürich (CH); Rast Christopher, San Diego CA 92106 (US); Aeschlimann Marcel, 2514 Ligerz (CH); Seiler Philipp, 4424 Arboldswil (CH)
(74) Representative: Maiwald Patentanwalts GmbH

(56) References cited:
- WO-A-02/069817
- WO-A-2004/017857
- JP-A- 2003 159 258
- US-A1- 2006 122 624

## Description

### BACKGROUND OF THE INVENTION

The invention relates in general to sonic fusion technology, it relating more particularly to a device for the fixation of bone fractures, with a bone screw for augmenting within a bone.

Known from US patent 4,653,489 is a system wherein a fixation cement is introduced through a bone screw into a portion of a bone afflicted by osteoporosis. Femoral neck fractures as well as distal femoral fractures can be fixated by means of this device.

Documents JP-A-2003/159 258 and WO-A-02/069 817 also disclose bone screws having a flow cavity.

The system in accordance with prior art comprises a bone screw having a flow cavity, i.e. an axial through bore through which bone cement can be introduced into the portion at the tip of the screw. The bone cement is advanced by a device which is releasably attached to the trailing end of the screw. This device is similar to a commercially available syringe in comprising substantially a cylindrical barrel and a plunger. The barrel forms a cavity in which the plunger is movable to and fro.

In use of this prior art device the fixation cement is filled into the barrel, after which the plunger is urged against the cement. By applying manual compression force the fixation cement is jetted into the axial through bore of the bone screw. Due to the pressure the fixation cement is adequately fluidized so that it can pass through the proximal end of the bone screw into the bone, as a result of which the bone screw is augmented in the bone.

This system has the drawback that the manual pressure applied to the fixation cement varies, not only basically from application to application but also during the application itself so that the distribution of the fixation cement within the portion of the bone at the tip of the bone screw is neither reliable nor even.

### SUMMARY OF THE INVENTION

An object of the invention is to define a device by means of which a reliable and even augmentation of a bone screw at an implantation site in the bone can be assured.

This is achieved by the subject matter of each independent claim. Further embodiments are described in the respective dependent claims.

Generally, a device for fixation of a bone fracture having a bone screw comprises a shank having a first threaded end and having along its longitudinal center line an axial through bore having a first bore portion with a first diameter and a second bore portion with a second diameter, wherein the first diameter is larger than the second diameter and the second bore portion is adjacent the first shank end, and a step in the bore between the first and the second bore portions.

Using the device for fixation of a bone fracture includes the steps of screwing a bone screw having an axial bore therein with transverse passageways connecting the bore with an outer surface of the bone screw into a bone across a fracture site; combining a polymer pin with a metal insert; inserting the polymer pin together with the metal insert into the axial through bore in the bone screw; pressuring and vibrating the polymer pin, wherein the polymer pin is supported by the metal insert which is in turn supported by a step in the diameter in the through bore, resulting in the polymer pin being fluidized at its tip, the fluidized polymer material being pressed out of the bone screw.

The invention will now be detailed by way of a preferred embodiment with reference to the attached drawings in which:

### BRIEF DESCRIPTION OF THE DRAWINGS

FIG. 1 is a section view of a bone screw in accordance with one embodiment of the invention;
FIG. 2 is a detail view of the tip of the bone screw as shown in FIG. 1;
FIG. 3 is a view of a polymer pin in accordance with one embodiment of the invention;
FIGS. 4a and 4b are a side view and respectively a plan view of a metal insert in accordance with one embodiment of the invention;
FIG. 5 is a section view of a device for fixation of a bone fracture in accordance with one embodiment of the invention, with the bone screw as shown in FIG. 1 in which the polymer pin as shown in FIG 3 and the metal insert as shown in FIGS. 4a, 4b are inserted; and
FIG. 6 is a detail view of the tip of the device as shown in FIG. 5.

### DETAILED DESCRIPTION

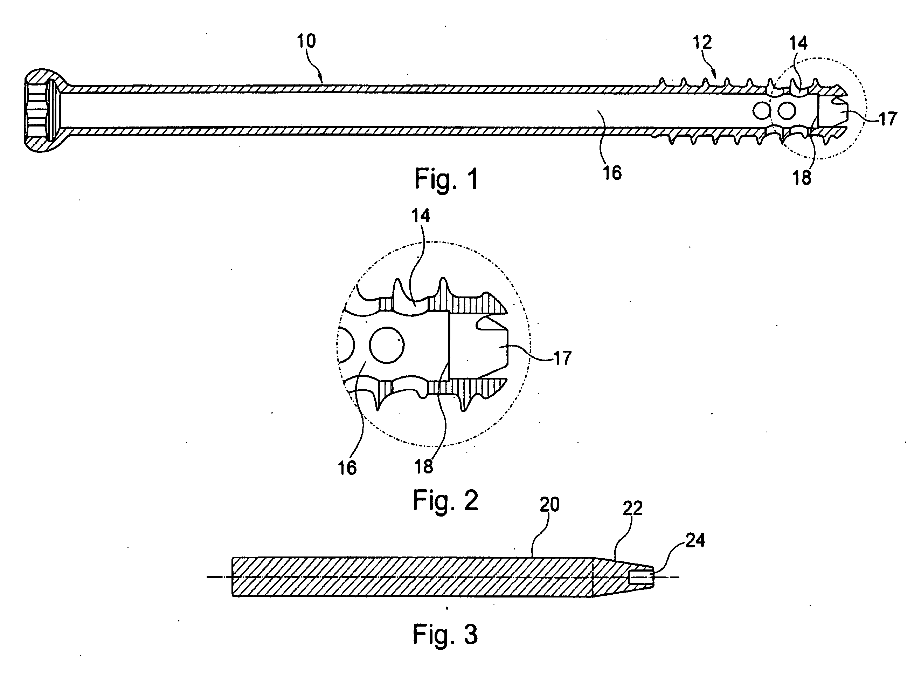

Referring now to FIG. 1 there is illustrated a bone screw 10 in accordance with the invention. The bone screw comprises a shank and a thread 12 machined in an end portion of the shank, although the thread may also cover the shank of the screw full length. In addition, the bone screw 10 is cannulated. The cannulation is provided along the longitudinal center line of the screw as a through bore 11 composed of two bore portions 16, 17. Bore portion 16 comprises a first diameter and bore portion 17 a second diameter, the first diameter being larger than the second diameter. In addition the bore portion 16 forms the main portion of through bore 11. In the preferred embodiment, just a small portion adjoining the end of the shank of the bone screw in which the thread 12 is machined is formed by the bore portion 17. The transition from the bore portion 16 to the bore portion 17 is formed by a step 18 in the bore 11. The step 18 in the bore forms an annular ridge having substantially right-angled edges at the wall of the through bore within the bone screw. The edges of the step 18 in the diameter may be machined flat or rounded. The screw features transverse holes 14 which extend through the wall of the bore portion 16 to allow polymeric material within the bore 11 to flow out of bore 11 and into the adjacent bore. Furthermore the position of the step in diameter together with the holes in the wall can be positioned optionally along the longitudinal center line and thus the location of the polymeric augmentation can be determined in accordance with the particular application and the desired effect.

In the preferred embodiment the holes 14 may be configured in differing directions perpendicular to the longitudinal center line of the bone screw and arranged in the end portion with the thread 12. Preferably the holes 14 are arranged in a region of the end portion which also features the bore portion 16. In the embodiment as shown in FIG. 1 two holes 14 each are configured axially juxtaposed in the bore portion 16 and through the thread 12. Furthermore, four such pairs of holes are evenly distributed about the circumference of the bone screw, in other words, circumferentially spaced by 90°. It is, however, just as possible that three, four, five or more holes may be provided circumferentially and it is not necessary that the holes circumferentially distributed are all at same axial level. Apart from this, transverse or longitudinal oblong holes, slots, or the like may be provided.

Referring now to FIG. 2 there is illustrated the tip of the bone screw as shown in FIG. 1 but on a magnified scale, the step 18 in the diameter between the bore portion 16 and bore portion 17 now being particularly evident. Apart from this, a few of the holes 14 are shown which are configured passing through the thread 12 in the bore portion 16.

Referring now to FIG. 3 there is illustrated a polymer pin 20 elongated in shape and slightly tapered at a conically tapered end 22. Provided in the conically tapered end 22 of the polymer pin 20 is a concavity or counterbore 24 in the end face. The polymer pin 20 may also be made of other materials such as for instance a thermoplastic material suitable for augmenting a bone screw, both resorptive and non-resorptive materials being useful.

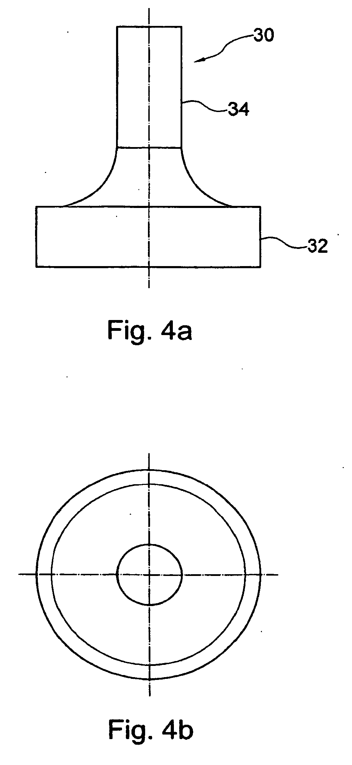

Referring now to FIGs. 4a and 4b a metal insert is shown in a side view and in a plan view. The metal insert features a substantially disk-shaped end 32 and a substantially pin-shaped end 34. The disk-shaped end 32 has an outer diameter somewhat smaller than the diameter of the bore portion 16 and somewhat larger than the diameter of the bore portion 17. The pin-shaped end 34 is configured so that it can be inserted into the counterbore in the polymer pin.

In another embodiment (not shown) the metal insert features instead of the pin-shaped end 34 a protruding end suitable for snap mounting, the polymer pin in this case having a snap mounting end corresponding to the protruding end. When the metal insert is snap mounted with the polymer pin, both elements can be inserted together into the bone screw, it being of advantage when the snap mount comprises a slight clearance when connected. This clearance has the advantage that when the polymer pin is pressurized it can be better fluidized at the joint with the metal insert to thus easier jetted from the bone screw into the bone easier.

It is furthermore possible that the metal insert instead of featuring a protruding or pin-shaped end has a through bore into which a corresponding end of the polymer pin can engage. In this embodiment the polymer material is jetted axially from the bone screw not only through the holes 14 but also out the leading end of the screw through the hole in the metal insert. the proportion of the polymer material emerging from the holes and bores can be varied by the size thereof.

Depending on the aspect concerned, a snap mount may also be provided in combination with axial and/or radial holes, it being just as possible, however, to configure the metal insert integrally with the bone screw. In this arrangement the step in the diameter between two portions of the bore is configured by a larger difference in diameter; indeed, even an axial blind hole may be used on the cannulation instead of the full axial through bore in the bone screw.

The following details inserting the bone screw into a bone. Firstly a K wire is powered up to the site in a bone at which the bone screw is to be located. Via the K wire the bone screw is then advanced and ultimately screwed into place until it is sited as desired. After insertion of the bone screw in the bone the K wire is removed. This procedure makes it necessary that the bone screw features a full length through bore. This is a popular operation technique because the operator can best check the position of the screw. The K wire is also used to measure the necessary screw length.

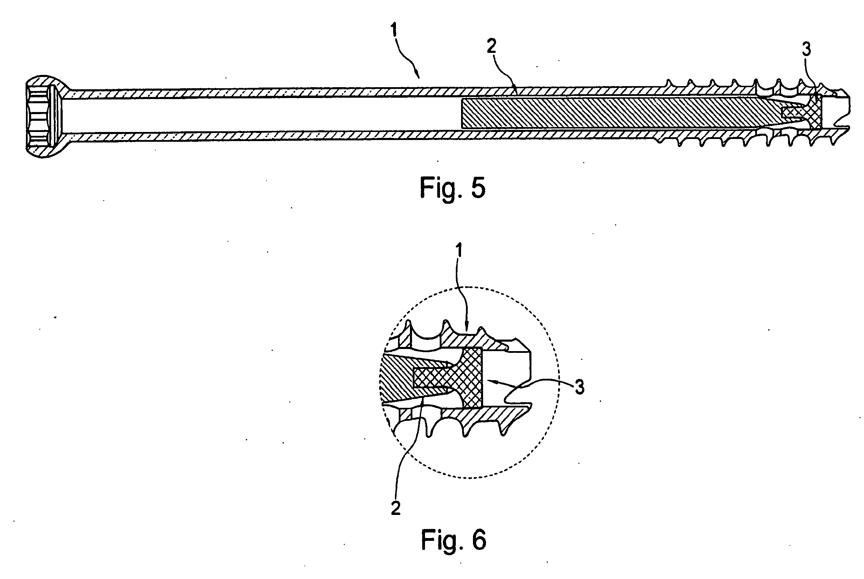

After removal of the K wire the passageway or through bore 11 along the longitudinal center line of the bone screw is free to receive polymer pin 20 together with metal insert 30. Tip 34 of the pin shaped end of metal 30 insert is inserted into counterbore 24 of polymer pin 20. Referring now to FIG. 5 there is illustrated how metal insert 30 rests on the step formed by the step 18 in the bore when polymer pin 20 with the metal insert 30 has been inserted facing the direction of the tip. It is in this way that the step 18 in the diameter forms within the bone screw a counterhold for the metal insert which in turn supports the polymer pin when the polymer pin is pressurized and vibrated by an ultrasonic handpiece/sonotrode which, for this purpose, is mounted on the free end of the bone screw. The vibration and pressure generated by the ultrasonic handpiece and applied to the polymer pin fluidizes the polymer pin so that the material of the polymer pin emerges from the radially arranged holes 14 into the bone. It is in this way that the polymer pin furnishes the material for augmenting the bone screw in the bone.

It is to be noted that the present invention is not just limited to the indications as recited above. In other words, all screw applications which can be supplied by cannulated screws can be potentially supplied with the option of polymeric fastening as defined in claims 1-3 and thus with the device in accordance with the invention.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. A device for fixation of a bone fracture, having a bone screw (10) comprising a shank having a first threaded end and having along its longitudinal centre line an axial through bore with transverse holes (14) connecting the bore with an outer surface of the bone screw, the axial through bore having a first bore portion (16) with a first diameter and a second bore portion (17) with a second diameter, wherein the first diameter is larger than the second diameter and the second bore portion is adjacent the first shank end, and a step (18) in the bore between the first and the second bore portions, wherein the device further comprises a metal insert (30) and a fluidizable polymer pin (20) for insertion into the bore of the bone screw, wherein the metal insert is substantially disk-shaped and dimensioned so that it can rest on the step in the bore when the metal insert is inserted into the bore of the bone screw, the metal insert having a pin-shaped end (34), and wherein a first end of the polymer pin has a recess, corresponding to the pin-shaped metal insert for receiving the same.

2. A device for fixation of a bone fracture, having a bone screw comprising a shank having a first threaded end and having along its longitudinal centre line an axial through bore having a first bore portion with a first diameter and a second bore portion with a second diameter, wherein the first diameter is larger than the second diameter and the second bore portion is adjacent the first shank end, and a step in the bore between the first and the second bore portions, wherein the device further comprises a metal insert and a fluidizable polymer pin for insertion into the bore of the bone screw, wherein the metal insert is substantially disk-shaped and dimensioned so that a first end rests on the step in the bore, when the metal insert is inserted into the bore in the bone screw and has a through bore, and wherein one end of the polymer pin is configured so that it is insertable into the through bore of the metal insert.

3. A device for fixation of a bone fracture, having a bone screw comprising a shank having a first threaded end and having along its longitudinal centre line an axial through bore with transverse holes (14) connecting the bore with an outer surface of the bone screw, the axial through bore having a first bore portion with a first diameter and a second bore portion with a second diameter, wherein the first diameter is larger than the second diameter and the second bore portion is adjacent the first shank end, and a step in the bore between the first and the second bore portions, wherein the device further comprises a metal insert and a fluidizable polymer pin for insertion into the bore of the bone screw, wherein the metal insert comprises a substantially disk-shaped first end dimensioned so that it rests on the step in the bore when the metal insert is inserted into the bone screw bore and has a first snap-mounting end, and wherein one end of the polymer pin has a second snap-mounting end configured to receive the first snap-mounting end on the metal insert with which it is connectable.

4. The device as set forth in any of claims 1 to 3, wherein the shank has a non-threaded portion and the step in the trough bore is located in said first threaded end.

5. The device as set forth in any of claims 1 to 3 wherein the bone screw further comprises holes (14) in a wall of the bore of the bone screw, the holes adjacent the step (18) in the bore.

## Patentansprüche

1. Vorrichtung zur Fixierung von Knochenbrüchen, die eine Knochenschraube (10) aufweist, die einen Schaft umfasst, der ein erstes Ende mit Gewinde aufweist und entlang seiner Längsmittellinie eine axiale Durchgangsbohrung mit Querlöchern (14) aufweist, die die Bohrung mit einer Außenfläche der Knochenschraube verbinden, wobei die axiale Durchgangsbohrung einen ersten Bohrungsabschnitt (16) mit einem ersten Durchmesser und einen zweiten Bohrungsabschnitt (17) mit einem zweiten Durchmesser, wobei der erste Durchmesser größer als ist der zweite Durchmesser und der zweite Bohrungsabschnitt dem ersten Schaftende benachbart ist, und eine Stufe (18) in der Bohrung zwischen dem ersten und dem zweiten Bohrungsabschnitt aufweist, wobei die Vorrichtung ferner einen Metalleinsatz (30) und einen fluidisierbaren Polymerstift (20) zum Einsetzen in die Bohrung der Knochenschraube umfasst, wobei der Metalleinsatz im Wesentlichen scheibenförmig und derart bemessen ist, dass er auf der Stufe in der Bohrung zur Anlage kommen kann, wenn der Metalleinsatz in die Bohrung der Knochenschraube eingesetzt ist, wobei der Metalleinsatz ein stiftförmiges Ende (34) aufweist, und wobei ein erstes Ende des Polymerstifts eine Aussparung aufweist, die zu dem stiftförmigen Metalleinsatz korrespondiert, um denselben aufzunehmen.

2. Vorrichtung zur Fixierung von Knochenbrüchen, die eine Knochenschraube aufweist, die einen Schaft umfasst, der ein erstes Ende mit Gewinde aufweist und entlang seiner Längsmittellinie eine axiale Durchgangsbohrung mit einem ersten Bohrungsabschnitt mit einem ersten Durchmesser und einem zweiten Bohrungsabschnitt mit einem zweiten Durchmesser, wobei der erste Durchmesser größer ist als der zweite Durchmesser und der zweite Bohrungsabschnitt dem ersten Schaftende benachbart ist, und eine Stufe in der Bohrung zwischen dem ersten und dem zweiten Bohrungsabschnitt aufweist, wobei die Vorrichtung ferner einen Metalleinsatz und einen fluidisierbaren Polymerstift zum Einsetzen in die Bohrung der Knochenschraube umfasst, wobei der Metalleinsatz im Wesentlichen scheibenförmig und derart bemessen ist, dass ein erstes Ende auf der Stufe in der Bohrung zur Anlage kommt, wenn der Metalleinsatz in die Bohrung in der Konchenschraube eingesetzt ist, und eine Durchgangsbohrung aufweist, und wobei ein Ende des Polymerstifts derart gestaltet ist, dass er in die Durchgangsbohrung des Metalleinsatzes einsetzbar ist.

3. Vorrichtung zur Fixierung von Knochenbrüchen, die eine Knochenschraube aufweist, die einen Schaft umfasst, der ein erstes Ende mit Gewinde aufweist und entlang seiner Längsmittellinie eine axiale Durchgangsbohrung mit Querlöchern aufweist, die die Bohrung mit einer Außenfläche der Knochenschraube verbinden, wobei die axiale Durchgangsbohrung einen ersten Bohrungsabschnitt mit einem ersten Durchmesser und einen zweiten Bohrungsabschnitt mit einem zweiten Durchmesser, wobei der erste Durchmesser größer ist als der zweite Durchmesser und der zweite Bohrungsabschnitt dem ersten Schaftende benachbart ist, und eine Stufe in der Bohrung zwischen dem ersten und dem zweiten Bohrungsabschnitt aufweist, wobei die Vorrichtung ferner einen Metalleinsatz und einen fluidisierbaren Polymerstift zum Einsetzen in die Bohrung der Knochenschraube umfasst, wobei der Metalleinsatz ein im Wesentlichen scheibenförmiges erstes Ende umfasst, das derart bemessen ist, dass es auf der Stufe in der Bohrung zur Anlage kommt, wenn der Metalleinsatz in die Knochenschraubenbohrung eingesetzt ist, und ein erstes Schnappbefestigungsende aufweist, und wobei ein Ende des Polymerstifts ein zweites Schnappbefestigungsende aufweist, das gestaltet ist, um das erste Schnappbefestigungsende auf dem Metalleinsatz, mit dem es verbunden werden kann, aufzunehmen.

4. Vorrichtung nach einem der Ansprüche 1 bis 3, wobei der Schaft einen Abschnitt ohne Gewinde aufweist und die Stufe in der Durchgangsbohrung im ersten Gewindeende angeordnet ist.

5. Vorrichtung nach einem der Ansprüche 1 bis 3, wobei die Knochenschraube ferner Löcher (14) in einer Wand der Bohrung der Knochenschraube umfasst, wobei die Löcher der Stufe (18) in der Bohrung benachbart sind.

## Revendications

1. Dispositif de fixation d'une fracture osseuse, ayant une vis à os (10) comportant une tige ayant une première extrémité filetée et ayant le long de son axe longitudinal, un alésage traversant axial muni de trous transversaux (14) reliant l'alésage à une surface extérieure de la vis à os, l'alésage traversant axial ayant une première partie d'alésage (16) avec un premier diamètre et une seconde partie d'alésage (17) avec un second diamètre, dans lequel le premier diamètre est plus grand que le second diamètre, et la seconde partie d'alésage est adjacente à la première extrémité de tige, et un épaulement (18) dans l'alésage entre les première et seconde parties d'alésage, dans lequel le dispositif comporte en outre un insert métallique (30) et une goupille en polymère fluidifiable (20) pour une insertion dans l'alésage de la vis à os, dans lequel l'insert métallique est sensiblement en forme de disque et dimensionné de sorte qu'il peut être en appui sur l'épaulement dans l'alésage lorsque l'insert métallique est inséré dans l'alésage de la vis à os, l'insert métallique ayant une extrémité en forme de goupille (34), et dans lequel une première extrémité de la goupille en polymère a un évidement, correspondant à l'insert métallique en forme de goupille, pour recevoir celui-ci.

2. Dispositif de fixation d'une fracture osseuse, ayant une vis à os comportant une tige ayant une première extrémité filetée et ayant le long de son axe longitudinal, un alésage traversant axial ayant une première partie d'alésage avec un premier diamètre et une seconde partie d'alésage avec un second diamètre, dans lequel le premier diamètre est plus grand que le second diamètre, et la seconde partie d'alésage est adjacente à la première extrémité de tige, et un épaulement dans l'alésage entre les première et seconde parties d'alésage, dans lequel le dispositif comporte en outre un insert métallique et une goupille en polymère fluidifiable pour une insertion dans l'alésage de la vis à os, dans lequel l'insert métallique est sensiblement en forme de disque et dimensionné de sorte qu'une première extrémité est en appui sur l'épaulement dans l'alésage lorsque l'insert métallique est inséré dans l'alésage dans la vis à os et a un alésage traversant, et dans lequel une extrémité de la goupille en polymère est configurée de sorte qu'elle peut être insérée dans l'alésage traversant de l'insert métallique.

3. Dispositif de fixation d'une fracture osseuse, ayant une vis à os comportant une tige ayant une première extrémité filetée et ayant le long de son axe longitudinal, un alésage traversant axial muni de trous transversaux reliant l'alésage à une surface extérieure de la vis à os, l'alésage traversant axial ayant une première partie d'alésage avec un premier diamètre et une seconde partie d'alésage avec un second diamètre, dans lequel le premier diamètre est plus grand que le second diamètre, et la seconde partie d'alésage est adjacente à la première extrémité de tige, et un épaulement dans l'alésage entre les première et seconde parties d'alésage, dans lequel le dispositif comporte en outre un insert métallique et une goupille en polymère fluidifiable pour une insertion dans l'alésage de la vis à os, dans lequel l'insert métallique comporte une première extrémité sensiblement en forme de disque, dimensionné de sorte qu'il est en appui sur l'épaulement dans l'alésage lorsque l'insert métallique est inséré dans l'alésage de vis à os et a une première extrémité de montage par encliquetage, et dans lequel une extrémité de la goupille en polymère a une seconde extrémité de montage par encliquetage configurée pour recevoir la première extrémité de montage par encliquetage sur l'insert métallique à laquelle elle peut être raccordée.

4. Dispositif tel qu'exposé dans l'une quelconque des revendications 1 à 3, dans lequel la tige a une partie non filetée et l'épaulement dans l'alésage traversant est situé dans ladite première extrémité filetée.

5. Dispositif tel qu'exposé dans l'une quelconque des revendications 1 à 3, dans lequel la vis à os comporte également des trous (14) dans une paroi de l'alésage de la vis à os, les trous étant adjacents à l'épaulement (18) dans l'alésage.
